# EUROPEAN PATENT SPECIFICATION

(11) **EP 0 674 713 B1**
(45) Date of publication and mention of the grant of the patent: **03.02.1999**
(21) Application number: 94904441.6
(22) Date of filing: 14.12.1993
(51) Int. Cl.: C12N 15/53, C12N 15/82, C12N 15/29, C12N 5/10, C12N 1/21, A01H 4/00

(54) **A DISEASE RESISTANCE GENE FROM MAIZE AS A SELECTABLE MARKER**
KRANKHEITRESISTENZGEN AUS MAIS ALS SELEKTIONSMARKER.
GENE DE RESISTANCE A UNE MALADIE DU MAIS UTILISE COMME MARQUEUR SELECTIONNABLE

(30) Priority: 15.12.1992 US 995658
(43) Date of publication of application: 04.10.1995
(73) Proprietor: PIONEER HI-BRED INTERNATIONAL, INC., Des Moines, Iowa 50309 (US)
(72) Inventor: BRIGGS, Steven, P., Des Moines, IA 50321 (US); JOHAL, Gurmukh, S., Urbandale, IA 50323 (US)
(74) Representative: Goldin, Douglas Michael
(86) International application number: US9312146
(87) International publication number: WO9413825

(56) References cited:
- SCIENCE vol. 258 , 6 November 1992 , LANCASTER, PA US pages 985 - 987 JOHAL, G.S., ET AL. 'Reductase activity encided by the HM1 disease resistance gene in maize'
- EMBL SEQUENCE DATABASE RELEASE 34. ACCESSION NO. L02540. 19 November 1992 JOHAL, G.S., ET AL. 'NADPH HC-toxin reductase'
- MOLECULAR AND GENERAL GENETICS vol. 219, no. 3 , November 1989 , BERLIN DE pages 492 - 494 ANZAI, H., ET AL. 'Transgenic tobacco resistant to a bacterial disaese by the detoxification of a pathogenic toxin'

## Description

### TECHNICAL FIELD

This invention relates to the isolation of a gene which controls resistance to both a fungus and a fungal disease toxin and its use as a selectable marker.

### BACKGROUND OF THE INVENTION

Disease resistance genes are defined as Mendelian factors that cosegregate with the resistance trait. The gene HM1, which controls resistance to Cochliobolus carbonum Nelson race 1 was among the first disease resistance genes to be described. The disease caused by C. carbonum race 1 can be devastating, resulting in yield losses of 80% or more due to plant death and grain mold. The dominant allele, Hm1, and the duplicate factor, Hm2 are the only disease resistance genes that are known to be fixed at a high frequency in maize germplasm.

Since the discovery of a race-specific compatibility factor that is produced by the fungus, the disease caused by C. carbonum race 1 has been the subject of detailed study. This compatibility factor permits the fungus to infect certain genotypes of maize that would otherwise be resistant. The role of HM1 in providing resistance to the fungus is a function of its ability to cause reduced sensitivity to the compatibility factor. Further studies established that the presence of the compatibility factor confers the same race-specificity (for hm1/hm1 corn) on the oat pathogen, Cochliobolus victoriae. The structure of this compatibility factor, HC-toxin, is known but the mode of action remains to be elucidated. Recently, an enzyme that inactivates HC-toxin has been identified in extracts from maize. The enzyme, HC-toxin reductase (HCTR), is detectable only in extracts from resistant (Hml) genotypes. This establishes HCTR activity as the biochemical phenotype of Hm1.

### DISCLOSURE OF THE INVENTION

It has now been determined that the Hml gene can be used in conjunction with HC-toxin in a selectable marker system for use in maize transformation. When the cloned gene is linked to appropriate regulatory sequences for expression in plant cells and cotransformed into maize cells along with another quantitative or qualitative trait which is not selectable, it confers upon transformants a resistance to HC-toxin by virtue of the production of HCTR. The cells can continue to grow on medium containing the isolated HC-toxin. Nontransformed cells do not express the HCTR and are rapidly killed by the toxin the pathogen produces. The net effect is a tissue culture containing only transformed cells which can then be regenerated by known methods to form transformed shoots and even whole plants.

Accordingly, the invention provides a method of identifying plant transformation using C. carbonum or the toxin produced by C. carbonum as a phytotoxic marker, comprising the steps of:
a) culturing cells or tissues from a selected target plant,
b) introducing into the cell or tissue culture at least one copy of an expression cassette containing a DNA sequence coding substantially solely for the Hm 1 gene of maize, operably linked to plant regulatory sequences which cause the expression of the DNA sequence in plant cells.
c) introducing C. carbonum or the toxin it produces into the cell or tissue culture and
d) identifying transformed cells as the surviving cells in the cell or tissue culture.

Preferably, in the expression cassette, the DNA sequence coding substantially solely for the Hm 1 gene of maize has at least 90% translational homology to the sequence of SEQUENCE I.D. No. 1 or SEQUENCE I.D. No. 2.

### Genetic Materials and Propagation

Mutator stocks used in this work were obtained from Dr. D. Robertson, Iowa State University. Ac/Ds stocks used were obtained from D. I. Greenblatt, University of Connecticut. The methodology of HM1 mutant isolation was performed in the manner previously published by one of us [S.P. Briggs, Curr. Top. Plant Biochem. Physiol., 6:59 (1987)].

Inbred HM1 allele designations are: Pr, hm1-1; K61, hm1-2; P8, Hm1-A; Pr1, Hm1-Pr1; B79, Hm1-B79; 4Co63, Hm1-4C063; Pioneer PHV12, Hm1-PHV12.

### Experimental Approach

Transposon-tagging is known to be a reliable tool for isolating genes from maize. The basic tenet of tranposon tagging is that DNA rearrangements (i.e., insertions or excisions) that are concomitant with genetic (phenotypic) changes define a causal relationship which identifies the rearranged DNA as the changed gene. Several independent events of this nature are accepted as proof of identity. As described herein, we have observed that 5 mutant alleles of HM1 are associated with insertions within a transcribed region (hm1-656::Mu1), hm1-1369::Mu3, Hm1-1062::dHbr, hm1-2355, hm1-1040::Spm), and 1 deletion allele Def(HM1)-1790 is associated with loss of the transcribed region. Finally, only the wild-type allele (Hm1-Pr1) produced a 1.3 kb mRNA.

The transposable element family Mutator is particularly effective for generating forward mutations. Germinal reversion of mutants back to wild-type is rare with Mutator-induced alleles. Somatic reversion has been observed, but the small somatic sectors typical of Mutator cannot be identified for traits that are not cell autonomous or are complex, such as disease lesion development. Therefore, to obtain multiple genetic events that were coincident with rearrangements of HM1, a strategy based upon independent forward mutations rather than reversions was used.

To identify co-segregation between HM1 and a restriction fragment, the segregating progeny were first classified by examining their DNA on Southern blots. The blots were hybridized with probes for RFLP loci that flank the locus; PI0200644 and PIO200044 (obtained from D. Grant, Pioneer Hi-Bred International, Inc.) map 5 cM proximal and distal to HM1, respectively (2; unpublished observations). Only progeny that inherited alleles at both loci from the same parent were used in the analysis. This approach permitted the identification of progeny which inherited the 10 cM block of chromosome 1 bounded by the RFLP loci. By grouping together progeny that inherited either the mutant allele, or the recessive tester allele with which the mutant allele was paired, and then comparing the two classes with each other, restriction fragments that were common to one progeny class and absent from the other were identified. This method established linkage between restriction fragments and the 10 cM block that contains HM1.

To directly determine the linkage between HM1 and a restriction fragment, linked fragments were cloned and used to prepare DNA hybridization probes. Probes were then hybridized to a Southern blot of DNA from 60 progeny of the backcross K61/Pr1 x K61. The backcross progeny were scored for inheritance of the HM1 alleles by inoculating them with conidia of the C. carbonum race 1 strain SB111, and for inheritance of the alternate alleles detected by the DNA probes. A comparison of the patterns of inheritance revealed the linkage relationships between the probes and HM1.

The RFLP loci described above, plus an RFLP locus that is detected by the probe NPI429, were used to identify the progenitor of the mutant alleles. We obtained NPI429 from T. Helentjaris, Native Plants, Inc.

### Isolation of DNA and blot hybridization

Total DNA from the leaf tissue of maize, Sorghum (Pioneer inbred P285), and Coix (Lachrymae jobi) and seedling tissue of Arabidopsis thaliana L. (Landsberg erecta) was isolated by the urea extraction method as described in S. L. Dellaporta, J. Wood, J.B. Hicks, Plant Mol Biol Rep, 1, 18 (1983). Southern blots were prepared as described by Athma et al, Genetics, 128, 163 (1991). For RFLP analysis, DNA was transferred to nylon membranes (MSI from Fisher) and the hybridizations were performed as described above but without the addition of formamide. Probes were made from gel-purified DNA fragments and labeled by random priming (Amersham). The Mu1 specific probes was an internal 650 bp Aval/BstEII fragment isolated from pA/B5; the plasmid was provided by L. Taylor, Stanford University. The Mu3 specific probe was the internal HindIII/XbaI fragment of a clone that was obtained from V. Chandler, University of Oregon. The Spm probes were pBxl and pXS2.3 obtained from K. Cone, Brookhaven National Laboratory.

### Isolation of RNA and Northern Blotting

Total RNA from 5 to 6 day-old etiolated seedlings was isolated by the guanidine thiocyanate method [P. Chomczynski and N. Sacchi, Anal Biochem 162, 156 (1987)]. Poly(A)+ RNA was enriched using the polyATtract mRNA isolation system of Promega. Samples (^{~}15 ug) of poly(A)+ RNA were denatured using formaldehyde, fractionated in a 1.3% agarose gel, and blotted onto Hybond-N by standard techniques ( ). DNA probes were radiolabeled by random priming and the blots were hybridized and washed as described.

### Genomic Cloning

DNA isolated from homozygous mutant seedlings was digested with SstI or XhoI and the appropriate DNA fragments (as judged from the Southern blots) were purified by preparative gel electrophoresis and electroelution into dialysis tubing. This purified DNA was ligated to preannealed SstI or XhoI cut arms from the bacteriophage vector λ sep6-lac5 obtained from R. Martienssen, Cold Spring Harbor Laboratory. Packaging into Gigapack Gold (Stratagene) and screening of the libraries were carried out according to the manufacturers instructions. All clones were subcloned into Bluescript SK+ (Stratagene) and maintained in the SURE strain (Stratagene) of E. coli.

### PCR (Polymerase Chain Reaction) and Sequence Analysis

For analysis of DNA from the resistant progeny of the hm1-656::Mu1/hm1-1369::Mu3 heterozygote, primers homologous to sequences on each side of the insertion sites were used for PCR amplication. The primer sequences were: 5' CTG CTC ATG ACT CAT ATC AGG CGG TAG C 3' AND 5' GAC CAG CCG ACG CAG CAG CCC CGC CTT C 3'. PCR conditions were as described by Perkin Elmer-Cetus, except that the reactions were performed in 20% glycerol. Reactions were heated to 94 C for 3 min, then cycled 40 times for 1 min at 94 C, 2 min at 65 C, and 2 min at 72 C; and finally extended for 15 min at 72C. The gel-purified PCR products were reamplified and directly sequenced using synthetic sequencing primers.

### HM1 Mutants Derived from Mutator Stocks

Several mutant alleles of HM1 were recovered from Mutator element stocks, 4 of which were selected for further study. Criteria for selection included DNA hybridization data that ruled out pollen contamination as the source of the mutant alleles and a low frequency of susceptible progeny appearing within a family. RFLP loci were used to distinguish between the mutant alleles and the standard recessive alleles with which they had been paired. Segregating progeny were classified for inheritance of mutant alleles by use of the RFLP probes.

### hm1-656::Mu1

Progeny produced by fertilizing plants of the genotype y wx gl1 Hm1-B79 Mutator (designated 81-82-9537 Mu² per se by D. Robertson) with pollen from the hybrid, K61/Pr, resulted in the recovery of 2 susceptible plants out of 253 progeny. Both susceptible plants were found to be carrying the mutant allele, hm1-656::Mu1, indicating that they arose as the result of a small somatic sector on the ear. Pollen from both plants was used to fertilize ears on the Pioneer inbred, PHV12.

When progeny from the cross hm1-656::Mu1/hm1-1 x Hm1-PHV12/Hm1-PHV12 were classified according to inheritance of the hm1-656::Mu1 allele (by using the RFLP loci), a pattern of hybridization with probe DNA from the Mu1 element was observed. A 3.2 kb SstI fragment co-segregated with the hm1-656::Mu1 allele. No exceptions to this were found in a sample of 92 progeny. The 3.2 kb fragment, which contained Mu1 plus some flanking DNA, was excised from a gel, cloned into the SstI site of λ sep6-lac5, subcloned into Bluescript SK+, restriction mapped, and used to prepare a probe from the DNA flanking the Mu1 insertion (designated *656).

The *656 probe was hybridized to a Southern blot of DNA from 4 different homozygous mutants. Classification using RFLP loci indicated that each of the mutant alleles was derived from the B79 inbred parent (Hm1-B79). The observation of polymorphisms showed that a DNA rearrangement took place in the cloned region concomitant with the generation of the mutations. With the enzyme used, hm1-1062::dHbr produces a fragment identical to B79. However, polymorphisms between hm1-1062::dHbr and B79 can be detected using other enzymes.

### hm1-1369::Mu3

The hm1-1369::Mu3 allele was recovered as a single susceptible plant out of 230 progeny. The progeny were produced by pollinating plants of the genotype y wx gl1 Hm1-B-79 Mutator (designated 81-82-9537 Mu² per se by Dr. Robertson) with the hybrid, K61/Pr (hm1-2/hm1-1).

The heterozygote, hm1-1369::Mu3/hm1-2, was self-pollinated and the progeny were classified using RFLP loci. Hybridization of a Mu3 probe revealed a co-segregating band. A 4.6 kb SstI fragment co-segregated with the hm1-1369::Mu3 allele. The signal intensity difference caused by 2 doses of the allele, when homozygous, permitted hm1-1369::Mu3 to be scored as a codominant marker, which increased the mapping resolution. No recombinants were observed in a sample of 60 progeny. The 4.6 kb fragment, which contained Mu3 plus flanking DNA, was excised from the gel and cloned into the SstI site of λ sep6-lac5 subcloned into Bluescript SK+, restriction mapped, and used to prepare a probe (designated *1369) from the DNA that flanked the Mu3 insertion.

The *1369 probe detected the same polymorphisms that had been revealed by the *656 probe, indicating that the Mu1 and Mu3 elements had inserted into the same restriction fragment. This conclusion was confirmed by comparison of the restriction maps of the 2 clones. An anomaly was revealed with SstI in which the hm1-656::Mu1 DNA gave a 3.2 kb fragment when hybridized with the *656 probe but a 1.0 kb fragment when the *1369 probe was used; other enzymes failed to detect this anomaly. DNA sequence analysis showed that the Mu1 element had created an SstI site upon insertion into HM1.

An effort was made to recover resistant progeny from the Mu alleles. The heterozygote, hm1-656::Mu1/hm1-1369::Mu3 was fertilized using pollen from the inbred Pr (hm1-1). From 500 progeny, 1 resistant plant was recovered. Examination of DNA from 10 susceptible siblings of the resistant plant confirmed that all three parental alleles were segregating as expected. Examination of DNA from the resistant plant revealed a progenitor-sized fragment from B79 (designated Hm1-B79R) plus the hm1-1 allele from Pr. This plant was self-pollinated. DNA from its progeny was examined using probes *1369, PIO200644, PIO200044, and NPI429. The same progeny were tested for susceptibility to infection by inoculating with SB111. The results confirmed that resistance was conferred by the Hm1-B79R allele. Homozygous Hm1-B79R progeny were identified. The site of insertion of Mu1/Mu3 was amplified by PCR and sequenced with no difference observed between the new allele and the B79 progenitor.

### hm1-1062::dHbr

Progeny produced by pollinating the hybrid, K61/Pr (hm1-2/hm1-1), with plants of the genotype y wx gl1 Hm1-B79 Mutator (designated 81-82-9539 Mu² per se by Dr. Robertson) resulted in the recovery of the hm1-1062::dHbr allele from 1 of 2 susceptible plants out of 483 progeny.

Hybridization with probes specific for Mu1, Mu3, Mu7, and Mu8 failed to identify a fragment that cosegregated with hm1-1062::dHbr. Hybridization with the *656 probe detected a polymorphism between hm1-1062::dHbr and the progenitor allele, Hm1-B79. We cloned a 3.1 kb XhoI fragment from the hm1-1062::dHbr mutant into λ sep6-lac5, using *656 as a probe. Restriction mapping confirmed the B79 structure with the exception of a small (approximately 400 bp) insertion into the SstI-XhoI fragment at the 3'-end of the clone. The identity of this insertion element has not been determined, but it lacks homology with Ac, Ds1, Ds2, and Spm/En internal sequence probes and with the Mu terminal inverted repeat.

### Def(HM1-1790)

Progeny produced by pollinating plants of the genotype y wx gl1 Hm1-B79 Mutator (designated 81-82-9536 Mu₂ per se by D. Robertson) with the hybrid, K61/Pr (hm1-2/hm1-1), resulted in the recovery of the Def(HM1)-1790 allele from a single susceptible plant out of 345 progeny.

The Def(HM1)-1790 allele displays an aberrant transmission pattern. A Def(HM1)-1790/hm1-1 heterozygote was self-pollinated and the progeny were characterized using the flanking RFLP loci. only 14 of 56 progeny were found to have inherited the Def(HM1)-1790 chromosome. When a heterozygote was fertilized using pollen from a wild-type stock (SB509), 8 of 25 progeny inherited the Def(HM1)-1790 chromosome. When the heterozygote was used as the pollen source to fertilize a wild-type inbred (W23), none of 32 progeny inherited the Def(HM1)-1790 chromosome. The results show that the Def(HM1)-1790 allele (or chromosome) was not transmitted through the pollen and was only poorly transmitted through the egg. Such a pattern of transmission is typical of a chromosomal deficiency. The *1369 probe did not hybridize with DNA from the Def(HM1)-1790 allele, confirming the presence of a deletion and showing that the cloned region lies within the deletion. Test crosses with br2, which maps within 0.1 cM of HM1, produced only wild-type progeny. Likewise, both PIO200644 and PIO200044 detected 2 alleles in progeny that inherited Def(HM1)-1790. Therefore, the deletion cannot encompass more than 5 cM of the chromosome, being bounded by br2 and one of the RFLP loci.

### An HM1 mutant derived from an Ac/Ds stock

An allele, Hm1-1040::Spm, was recovered from the inbred 4Co63; the P-VV allele (Ac inserted into the P1 gene) had been backcrossed into this version of 4Co63. This allele was selected for study because it appeared to arise as a large tassel sector. The hybrid K61/Pr (hm1-2/hm1-1) was fertilized using pollen from the P-VV/P-WW) inbred 4Co63 (Hm1-4Co63/Hm1-4Co63), designated 610417(X) by I. Greenblatt (personal communication). Seventeen males were used to produce 32 ears. only 2 ears bore susceptible progeny and both were derived from male plant number 16. The cross 741-11 x 741-9 plant 16 yielded 47 susceptible progeny out of 323. The cross 741-13 X 741-9 plant 16 yielded 16 susceptible progeny out of 323. The results are best explained by a single mutagenic event that occurred during tassel development, giving rise to many gametes bearing the same mutation.

Genetic tests showed that Ac had not transposed in the hm1-1040::dSpm mutant; Ac, Ds1, and Ds2 probes failed to identify a restriction fragment that cosegregated with hm1-1040::dSpm. Examination of DNA from the hm1-1040::dSpm mutant by hybridization with the *1369 probe revealed an 11.0 kb SstI restriction fragment that was 6 kb larger than the Hm1-4Co63 progenitor allele. This fragment was cloned into λ sep6-lac5. Hybridization of various probes to DNA from the clone identified the insertion as an Spm/En homologous element.

### Natural Variation

The Hm1 parental alleles from which the mutants described in this report were derived provide complete resistance to C. carbonum race 1 throughout the development of the plant. In contrast, the Hm1-A allele, found in the inbred P8, provides partial resistance that increases as the plant develops. Examination of DNA from P8 by hybridization with the *1369 probe showed that the cloned region is duplicated. The duplicate locus segregates independently of HM1. The relationship between the pattern of expression and the duplication has not been established. The *1369 probe hybridized well with DNA from the grasses Sorghum and Coix but poorly with DNA from Arabidopsis. Poly(A)+ RNA from our mutants and from the inbreds K61 (hm1-2) and Prl (Hm1-Pr1) was blotted and hybridized with the *1062 probe. A 1.3 kb mRNA band was present only in the Prl lanes. The susceptible genotypes either had no detectable hybridizing mRNA or the size was aberrant. In all cases, the signal was extremely weak.

These results establish that all or a sufficient part of the HM1 gene has been cloned and sequenced, with genomic and cDNA sequences as shown in SEQUENCE I.D. No. 1 and SEQUENCE I.D. No. 2, respectively. Recessive alleles of the gene clearly contain homologous DNA (e.g., hm1-1 and hm1-2). This stands in contrast to the 2 fungal plant pathogen genes that have been cloned which control race specificity:: TOX2 confers race 1 type pathogenicity upon C. carbonum (Walton, personal communication) and avr9 confers race specific avirulence upon Cladosporium fulvum; both genes are missing in strains that lack their corresponding functions.

Resistance appears to differ from susceptible at the transcriptional level, at least among the small samples of alleles that were examined. This indicates that susceptible genotypes do not possess an alternative form of HM1 with specificity for a substrate other than HC-toxin. This result also suggests that the only function of HM1 in young leaf tissue is to provide resistance, since the HM1 mutations are not pleiotropic.

## Claims

1. A method of identifying plant transformation using resistance to C. carbonum or the toxin produced by C. carbonum as a phytotoxic marker, comprising the steps of:
a) culturing cells or tissues from a selected target plant,
b) introducing into the cell or tissue culture at least one copy of an expression cassette containing a DNA sequence coding substantially solely for the Hm 1 gene of maize, operably linked to plant regulatory sequences which cause the expression of the DNA sequence in plant cells.
c) introducing C. carbonum or the toxin it produces into the cell or tissue culture and
d) identifying transformed cells as the surviving cells in the cell or tissue culture.

2. A method according to claim 1 wherein, in the expression cassette, the DNA sequence coding substantially solely for the Hm 1 gene of maize has at least 90% translational homology to the sequence of SEQUENCE I.D. No. 1 or SEQUENCE I.D. No. 2.

## Patentansprüche

1. Verfahren zum Identifizieren einer Pflanzentransformation unter Verwendung einer Resistenz gegen C. carbonum oder gegen das von C. carbonum hergestellte Toxin als phytotoxischem Marker, welches die folgenden Stufen umfaßt:
a) Kultivieren von Zellen oder Geweben von einer ausgewählten Zielpflanze,
b) Einführen wenigstens einer Kopie einer Expressionskassette, die eine DNA-Sequenz enthält, die für im wesentlichen ausschließlich das Hm-1-Gen von Mais codiert, und die funktionell mit pflanzenregulatorischen Sequenzen, die die Expression der DNA-Sequenz in Pflanzenzellen bewirkt, verbunden ist, in die Zell- oder Gewebekultur,
c) Einführen von C. carbonum oder dem Toxin, das es herstellt, in die Zell- oder Gewebekultur und
d) Identifizieren transformierter Zellen als überlebende Zellen in der Zell- oder Gewebekultur.

2. Verfahren nach Anspruch 1, dadurch **gekennzeichnet**, daß in der Expresssionskassette die DNA-Sequenz, die im wesentlichen ausschließlich für das Hm-1-Gen von Mais codiert, eine wenigstens 90% translationelle Homologie zu der Sequenz der SEQUENZ I.D. No. 1 oder SEQUENZ I.D. No. 2 besitzt.

## Revendications

1. Une méthode destinée à identifier la transformation de plantes en utilisant la résistance à C. carbonum ou à la toxine produite par C. carbonum comme marqueur phytotoxique, comprenant les étapes consistant à :
a) cultiver les cellules ou les tissus provenant d'une plante cible choisie,
b) introduire dans la culture de cellules ou de tissus, au moins une copie d'une cassette d'expression contenant une séquence d'ADN codant substantiellement uniquement pour le gène Hm 1 du maïs, liée de manière fonctionnelle à des séquences régulatrices de plantes qui provoquent l'expression de la séquence d'ADN dans les cellules végétales.
c) introduire C. carbonum ou la toxine qu'il produit dans la culture de cellules ou de tissus et
d) identifier les cellules transformées en tant que cellules survivantes dans la culture de cellules ou de tissus.

2. Une méthode selon la revendication 1 dans laquelle, dans la cassette d'expression, la séquence d'ADN codant substantiellement uniquement pour le gène Hm 1 du maïs présente une homologie traductionnelle d'au moins 90 % avec la séquence de SEQUENCE I.D. N° 1 ou de SEQUENCE I.D. N° 2.


### SEQUENCE LISTING

### (1) GENERAL INFORMATION:

(i) APPLICANT: Briggs, Steven P.; Johal, Gurmukh S.
(ii) TITLE OF INVENTION: A Disease Resistance Gene from Maize as a Selectable Marker
(iii) NUMBER OF SEQUENCES: 2
(iv) CORRESPONDENCE ADDRESS:
   (A) ADDRESSEE: Pioneer Hi-Bred International, Inc.
   (B) STREET: 700 Capital Square, 400 Locust Street
   (C) CITY: Des Moines
   (D) STATE: Iowa
   (E) COUNTRY: United States
   (F) ZIP: 50309
(v) COMPUTER READABLE FORM:
   (A) MEDIUM TYPE: Diskette, 3.5 inch, 1.44 Mb storage
   (B) COMPUTER: IBM Compatible
   (C) OPERATING SYSTEM: MS-DOS
   (D) SOFTWARE: Microsoft Windows Notepad
(vi) CURRENT APPLICATION DATA:
   (A) APPLICATION NUMBER:
   (B) FILING DATE:
   (C) CLASSIFICATION:
(vii) PRIOR APPLICATION DATA:
   (A) APPLICATION NUMBER:
   (B) FILING DATE:
(viii) ATTORNEY/AGENT INFORMATION:
   (A) NAME: Roth, Michael J.
   (B) REGISTRATION NUMBER: 29,342
   (C) REFERENCE/DOCKET NUMBER: 0212 US
(ix) TELECOMMUNICATION INFORMATION:
   (A) TELEPHONE: (515) 245-3594
   (B) TELEFAX: (515) 245-3634

### (2) INFORMATION FOR SEQ ID NO: 1:

(i) SEQUENCE CHARACTERISTICS:
   (A) LENGTH: 5198 base pairs
   (B) TYPE: nucleotide
   (C) STRANDEDNESS: double
   (D) TOPOLOGY: linear
(ii) MOLECULE TYPE: genomic DNA
   (A) DESCRIPTION:
(iii) HYPOTHETICAL: No
(iv) ANTI-SENSE: NO
(v) FRAGMENT TYPE :
(vi) ORIGINAL SOURCE:
   (A) ORGANISM: Zea mays
   (B) STRAIN:
   (C) INDIVIDUAL ISOLATE:
   (D) DEVELOPMENTAL STAGE:
   (E) HAPLOTYPE:
   (F) TISSUE TYPE:
   (G) CELL TYPE:
   (H) CELL LINE:
   (I) ORGANELLE:
(vii) IMMEDIATE SOURCE:
   (A) LIBRARY:
   (B) CLONE:
(viii) POSITION IN GENOME:
   (A) CHROMOSOME/SEGMENT:
   (B) MAP POSITION:
   (C) UNITS:
(ix) FEATURE:
   (A) NAME/KEY:
   (B) LOCATION:
   (C) IDENTIFICATION METHOD:
   (D) OTHER INFORMATION:
(x) PUBLICATION INFORMATION:
   (A) AUTHORS:
   (B) TITLE:
   (C) JOURNAL:
   (D) VOLUME:
   (E) ISSUE:
   (F) PAGES:
   (G) DATE:
   (H) DOCUMENT NUMBER:
   (I) FILING DATE:
   (J) PUBLICATION DATE:
   (K) RELEVANT RESIDUES IN SEQ ID NO:
(xi) SEQUENCE DESCRIPTION: SEQ ID NO: 1:
   GAATTCGTAT ATCAGTTTAC TGCATGTATA TTTTTTGCAT GCACATTGGA 50
   AAGACAATTC CTTGATTTAT GTTGTTCGTA ATTACCAAAA AATCAAATCA 100
   TTTATGTTCG TAATTGCTAG ATTTTTACGT CTTCCATAAA ACTGTCCCTA 150
   ATTCTCGCCT GTTCTTAATT ACCATAAAAA ATCAAATCAA ATCACTTATA 200
   GTCTTAACTG CCAAATTTTT ATATCTGCCA TAAAATTGTC CCTAATTTCC 250
   GTCGGCGTTC TTAGTTGGAC CGATATTAGG TTTTTTCAAT TATGCTACAC 300
   TGTCTAAATA TTTATGCGAC GTTGTATAAG AATTTGTGCT TTGTGTGACT 350
   CATGTCATCC AATTTTTTGT GCGCGTGCAG TGGAAAGGAC ATATAATCTA 400
   AAATTTGCGT GCATGCAATG GAAACTCCTA CAATTTGCCT TAATTTTTAG 450
   ATGTGCATAA AAATAGAAAT TGCATGCATA CGACTCCCAT ATTTTTCAGA 500
   TCTGCCATAA AAATAGGATC GTAAATACAA CCCACCAAAC TATCAACCTC 550
   TCATATTGGC TCGATATTTA TGTTTATAAC TGAGAGGTTT TATGCCTAAA 600
   ACTTAAGGTA TTTACGCTCG AACCTGTAGA TGCATCCACC AGTGAACCAC 650
   ATGCCAGATT TTTTACGTAG TATACCACAT TGCATATATA ACTACACCAT 700
   GTTGCATAAT TTGTATAAGT ATATATCATA AACTGGTGCT AACGAGCCTA 750
   GTTGCATGTC TGTTGAAGCG ATCATATATT TATACACGAA ATAAAGCATT 800
   TAAATAATTT TTTTTTGTTT TCATGCATGC CAATCCATTT CCTTTCATCG 850
   CACATCCTGC CATCCTAAAT TTGTGTGCAT GCATCAAGAA ACAGATTTTT 900
   ACACAGTATA CTGAATTGAA TAAATACCTA CACCTTGTAA CATAATTAAT 950
   ATCAGTATAT TATAAACTAG TCCTAACGAA TTTAGTTGTA TGGCTGTTGG 1000
   AGCGATCCTA TATTTATGGA GAAAATAAAG TATTAAATAT TTTTTTTTGT 1050
   TTCCATGCAT GCCAATCCAT TTCCTTTCAT CGCATATTCT TGTCATCTTA 1100
   AATTTGTGCT CATGCAACAG GAAACAAATT TTTACGCAGT GTACCAAATT 1150
   GCATAAACAC CTACACCGTG TTGCATAATT AGTATCGTTA TATAACATAA 1200
   ACTAGTCTTG ACAAGCCTAT CTAAATGGGT TTTAGAGTGA TCTTATATTT 1250
   ATGGAGGAAA TAAAGCATTA AATATGAATA TTTGTTTTCA TACATGACAA 1300
   TTCATTTCCT TTTATCGTAC AAATTTTGCC ATCCTAATTT TGTGCACATG 1350
   CAGCAGGTAA AAAACTTTTA CGTAGTGTAC CAAATTGCAT AAATATCTAC 1400
   ACCGTGTAGC ATATTTAGTA TTAGTATATA TATCATAAAC TGGTCCTAAC 1450
   GAGCCTAGCT GCATAGCTGT TGCAGCGATC CTATATTTAT GGAGAAAATG 1500
   AATCATTTAA AAATAGAAAA AGTCAGAATC TTTTTTTTAA AACTTTACGC 1550
   GTATGCAGGG ACACATGTTT GACTTATGCA TCCATTCTTT TTTTGCGAGC 1600
   ATAAACGTGG GGTGGGGAGC GCACCCGACA AACTGGCTTG GGGAGTGAGT 1650
   GGGCGCGCCG GCCTGGACCA GGTCGCAGGG GTGGTCGGCC TGGGCTTCCT 1700
   CTAACTATTT GAAGCCCAGG GTTCCTAATA TACCTTTCCC TATATATATA 1750
   TGGCTTCTTT GGAAGCTTTA CTTAAAAAAC TTTAGCTTTA GCTTTTGAGC 1800
   TTCACCATAA AACAACTCCA GCAAATTGTT AAGGTGGGTT TCAGAAGCGT 1850
   TTGACTTCCA CATGAACTCC AGCTTCTATA GTACAATTGA TTTGTGTGAG 1900
   TTTCCTTGAT TACCCTTTAT GATTAGTGGG TTGTCGCGAG AGAAAACGTA 1950
   AGTTATATAT TGCGTAACCA GTGGCATGGT GGGTAATTTT TGTACAACTT 2000
   CATGAGGAGA TATAAAAACG GTGTTTTTGT AGCACCCTCT AAGAGTTTCA 2050
   TGAATTTCAT GAAATTGACC TCTAGTTTCG ATTTTTTTAA AGCTAGAGCT 2100
   CGTGGAGCTG GACCAGTTTA GGTGGAAGAA GTTGGAATGA AGTTGAAATT 2150
   TTTGAAGTGA AGCTCTCTCA AATAGAGCGT TAATCTACAT TATTAATATT 2200
   TGACTTCTAG ATAAGCGAGA ATGGCACTCT TTAGAATGGA GCAACTGGGC 2250
   TTAGCTACAT TTATGATATG GAGGTTCTTA GTTGTATTAT GTAGTTTCTT 2300
   AGCTACCTTT TATATTTGGA AAACCGAACC AGACTCTGGA TTGTACATAG 2350
   CCTTTGAAGA ATTATTCAAG AGTTAATTAA GCTGTAATGC CACTAATATT 2400
   AAGGTTTAAT TTACCCTTGT ATTCAATACT TAACGTTATA TATTCTCTAT 2450
   AATTAAACAT AGTCCAACTT AAAAAATGAT TTTTGCAAAG ATAGAATTAT 2500
   ATGTTTTTTG TGAGGGCTAA TAGTTTAATG AAGTTATCGA TGTTTGTATA 2550
   TTTCACGACT ATAACCTTCA CCTCTGACCG CTAATGCTTG TTTAGACCAT 2600
   ATTTGGATGT TGTTGTATTA AAATATTATG TTATTAAGGA ACCATTGTTT 2650
   TAGCATTGTA TTCAAAATTA TGAAGTTTTT ATATGCAAAT GATTCACTCG 2700
   AAAAAACAAA AGTATCAAAA TCATAGTATT TTCTCTCTCT AAAATTCCAA 2750
   AAATATTTTA CATCTAAAGT TGATGTCTTG GCCTCCCGCG CTGTTTTACC 2800
   TTCCTCCTCT GGACACCAAC CACAGGCCGA CAACCGAGTA AGCCGGTCAA 2850
   TTTTGGTATC CTGCTCATGA CTCATATCAG GCGGTAGCCG AGCAGCCGCC 2900
   CAGCTTTCTC ATGCCAGAGC AAACCCATAG GTCCAGTCCA AATCCAATCC 2950
   CTGTTGCCAT CAGAATTTCA GGGGCAGCCA TGGCCGAAAA GGAGAGCAAC 3000
   GGAGTGCGGG TGTGCGTCAC CGGAGGAGCC GGGTTCATCG GCTCCTGGCT 3050
   CGTCAGGAAG CTCCTCGAGA AAGGCTACAC CGTCCACGCC ACCCTGCGGA 3100
   ACACCGGTGC GTCTGATGGC GGCTCCTCAG CTCGATCCGC GCGTCGCGAA 3150
   AGGCGAAACA CGCCAAAGGC GAAAGGAGTG GGTCGGGTCT CGTGTGTGGC 3200
   TGCCTGATGA TTCGGGAATC TTAGCCGGAT TCGTGTGCTG GTGGGTGTGC 3250
   AGGGGACGAG GCGAAGGCGG GGCTGCTGCG TCGGCTGGTC CCCGGCGCGG 3300
   CGGAGCGGCT GCGGTTGTTC CAGGCCGACC TCTTCGACGC CGCCACCTTC 3350
   GCGCCGGCGA TCGCTGGGTG CCAGTTCGTC TTCCTCGTCG CCACGCCATT 3400
   CGGGCTCGAT AGCGCCGGCT CCCAGGTGAA GCTTGCCGTC GCGTTCGCTC 3450
   CCTTCACTGT TTTACTAGTC AACGAGTGGT CAGGCGGCGA CGCGCCGTGC 3500
   TGTCTCTTCT TAATTTTAAG TTGTGGAAAA TTACTGTCCT TGCAAAGGAA 3550
   AAATTTGATC AGACTGAGTA TGAGTAAAGA CCAGTAAGAC AGCACAAGGA 3600
   TTGCGAGCTG CGACTGCGAG CAGAGGAAAC GTCTTCACAG TTATTCTTGT 3650
   CTGCCTTGTT TTTCTCTATC TGAAGCAAAA TCTTGTGGTG CCCATCGACC 3700
   GCGTATAGCA GTATAAGAGC ACGGCGGAAG CTGTGGTGGA CGCGGTGCGC 3750
   GCGATCCTCC GGCAATGCGA GGAGTCCCGG ACGGTGAAGC GAGTGATCCA 3800
   CACAGCCTCC GTAGCGGCCG CCTCGCCGTT GCTGGAGGAG GAGGTCTCCG 3850
   CCTCCGGCGT CGGGTACAGA GACTTCATCG ACGAATCTTG TTGGACTTCG 3900
   CTCAACGTTG ACTATCCTCT CCGAAGCGCA CACTTCGACG TAAGTAGTAT 3950
   ACAAGCGAAG CTTCTTCTGA TTTCTGAACT GGAACGCCTG ATCACATTAA 4000
   TATTTTTTAG CTGACGGCCA TTTGATTTGC AGAAGTACAT ACTGTCGAAG 4050
   CTGCGGTCAG AGCAGGAGCT CCTGAGCTAC AACGGCGGCG AGAGCCCGGC 4100
   GTTCGAGGTG GTGACCCTGC CGCTGGGGCT CGTGGCGGGC GACACGGTCC 4150
   TCGGCCGCGC CCCGGAGACG GTGGAGAGCG CCGTGGCGCC CGTGTCCCGC 4200
   AGCGAGCCCT GCTTCGGCCT CCTGCGCATA CTGCAGCAGC TCCTGGGGTC 4250
   GCTGCCGCTG GTGCACGTGG ACGACGTCTG CGACGCGCTC GTCTTCTGCA 4300
   TGGAGCGGCG CCCCTCCGTC GCCGGCCGCT TCCTCTGCGC CGCCGCGTAC 4350
   CCGACGATCC ACGACGTGGT CGCCCACTAC GCCAGCAAGT TCCCTCACCT 4400
   CGACATCTTG AAAGAGTAAG ATCAAAAGCG TCCACAGCGA CAGCATCACC 4450
   CTGCACACAA GAACTGACTG CCGATTTACG TTTCTGTTGC GATTGGTTGG 4500
   ATTGATCTGC GTCAGGACGG AGGCGGTGGC GACGGTGCGG CCTGCCCGGG 4550
   ACAGGTTGGG CGAGCTGGGC TTCAAGTACA AGTACGGCAT GGAAGAGATT 4600
   CTGGATAGCA GCGTTGCCTG TGCGGCGAGA TTAGGTTCCC TTGACGCATC 4650
   CAAGCTCGGC CTACAGAAAG GATAAAAGCT CGAAGCTTAC TCATAAGCAC 4700
   CATGGGGAAC TTGGATTGTT CGCTGTCCAC TATACGCGTT CGAAATTTGG 4750
   AAACTAGACA TACTCCAATA AAACAAGAGG TAAAGAAACG TGGGCTAACT 4800
   GATACGCGTT GAGCAGTTGA GCTAGCCTAG TTTAGTCCAC CTGTGTGCAG 4850
   GGTTTAAAAC TTCGACGAAA TTTTATGACT TGCGATAATT TTAGGCCTCT 4900
   AAATATCAAC CATACACTCT AAATTGTATA TGTGCATACA CATATAGCCA 4950
   TATGGACGCT CTGATCTAGC ATCCTACACC TATGCACCTC TCTAAGAACA 5000
   ACTCCAACAG CCTCACTAAA TATATCAGAT TCGGTAAAAA AAAACCCAGT 5050
   TAAAATTGTA TCCAATAGTC TCGTTTTATT CTTATCTTCT CTATCCAACT 5100
   CGTCATATGG TCCCTTTCGC TAGACATCTT TGCCAAGGCG TACGGCTCGC 5150
   CATATCCCTC GTCATGCCCA ACTGTCCTCC CGCCGTCGCA GAGAATTC   5198

### (2) INFORMATION FOR SEQ ID NO: 2:

(i) SEQUENCE CHARACTERISTICS:
   (A) LENGTH: 1374 base pairs
   (B) TYPE: nucleotide
   (C) STRANDEDNESS: double
   (D) TOPOLOGY: linear
(ii) MOLECULE TYPE: cDNA
   (A) DESCRIPTION:
(iii) HYPOTHETICAL: No
(iv) ANTI-SENSE: NO
(v) FRAGMENT TYPE :
(vi) ORIGINAL SOURCE:
   (A) ORGANISM: Zea mays
   (B) STRAIN:
   (C) INDIVIDUAL ISOLATE:
   (D) DEVELOPMENTAL STAGE:
   (E) HAPLOTYPE:
   (F) TISSUE TYPE:
   (G) CELL TYPE:
   (H) CELL LINE:
   (I) ORGANELLE:
(vii) IMMEDIATE SOURCE:
   (A) LIBRARY:
   (B) CLONE:
(viii) POSITION IN GENOME:
   (A) CHROMOSOME/SEGMENT:
   (B) MAP POSITION:
   (C) UNITS:
(ix) FEATURE:
   (A) NAME/KEY:
   (B) LOCATION:
   (C) IDENTIFICATION METHOD:
   (D) OTHER INFORMATION:
(x) PUBLICATION INFORMATION:
   (A) AUTHORS:
   (B) TITLE:
   (C) JOURNAL:
   (D) VOLUME:
   (E) ISSUE:
   (F) PAGES:
   (G) DATE:
   (H) DOCUMENT NUMBER:
   (I) FILING DATE:
   (J) PUBLICATION DATE:
   (K) RELEVANT RESIDUES IN SEQ ID NO:
(xi) SEQUENCE DESCRIPTION: SEQ ID NO: 2:
   GAATTCGGCA CGAGTGCCAT CAGAATTTCA GGGGCAGCCA TGGCCGAAAA   50
   GGAGAGCAAC GGAGTGCGGG TGTGCGTCAC CGGAGGAGCC GGGTTCATCG  100
   GCTCCTGGCT CGTCAGGAAG CTCCTCGAGA AAGGCTACAC CGTCCACGCC  150
   ACCCTGCGGA ACACCGGGGA CGAGGCGAAG GCGGGGCTGC TGCGTCGGCT  200
   GGTCCCCGGC GCGGCGGAGC GGCTGCGGTT GTTCCAGGCC GACCTCTTCG  250
   ACGCCGCCAC CTTCGCGCCG GCGATCGCTG GGTGCCAGTT CGTCTTCCTC  300
   GTCGCCACGC CATTCGGGCT CGATAGCGCC GGCTCCCAGT ATAAGAGCAC  350
   GGCGGAAGCT GTGGTGGACG CGGTGCGCGC GATCCTCCGG CAATGCGAGG  400
   AGTCCCGGAC GGTGAAGCGA GTGATCCACA CAGCCTCCGT AGCGGCCGCC  450
   TCGCCGTTGC TGGAGGAGGA GGTCTCCGCC TCCGGCGTCG GGTACAGAGA  500
   CTTCATCGAC GAATCTTGTT GGACTTCGCT CAACGTTGAC TATCCTCTCC  550
   GAAGCGCACA CTTCGACAAG TACATACTGT CGAAGCTGCG GTCAGAGCAG  600
   GAGCTCCTGA GCTACAACGG CGGCGAGAGC CCGGCGTTCG AGGTGGTGAC  650
   CCTGCCGCTG GGGCTCGTGG CGGGCGACAC GGTCCTCGGC CGCGCCCCGG  700
   AGACGGTGGA GAGCGCCGTG GCGCCCGTGT CCCGCAGCGA GCCCTGCTTC  750
   GGCCTCCTGC GCATACTGCA GCAGCTCCTG GGGTCGCTGC CGCTGGTGCA  800
   CGTGGACGAC GTCTGCGACG CGCTCGTCTT CTGCATGGAG CGGCGCCCCT  850
   CCGTCGCCGG CCGCTTCCTC TGCGCCGCCG CGTACCCGAC GATCCACGAC  900
   GTGGTCGCCC ACTACGCCAG CAAGTTCCCT CACCTCGACA TCTTGAAAGA  950
   GACGGAGGCG GTGGCGACGG TGCGGCCTGC CCGGGACAGG TTGGGCGAGC 1000
   TGGGCTTCCA AGTACCAAGT ACGGCATGGG AAGAGATTCT GGATAGCAGC 1050
   GTTGCCTGTG CGGCGAGATT AGGTTCCCTT GACGCATCCA AGCTCGGCCT 1100
   ACAGAAAGGA TAAAAGCTCG AAGCTTACTC ATAAGCACCA TGGGGAACTT 1150
   GGATTGTTCG CTGTCCACTA AACGCGTTCG AAATTTGGAA ACTAGACATA 1200
   CTCCAATAAA ACAAGAGGTA AAGAAACGTG GGCTAACTGA TACGCGTTGA 1250
   GCAGTTGAGC TAGCCTAGTT TAGTCCACCT GTGTGCAGGG TTTAAAACTT 1300
   CGACGAAATT TTATGACTTG CGATAATTTT AGGCCTCTAA AAAAAAAAAA 1350
   AAAAAAAAAA AAAAAAAACT CGAG                             1374